(19) Europäisches Patentamt
European Patent Office
Office européen des brevets

(11) Publication number: 0 195 498 A2

# (12) EUROPEAN PATENT APPLICATION

(21) Application number: 86300582.3

(22) Date of filing: 29.01.86

(51) Int. Cl.4: C07D 451/02 , C01B 33/28

(30) Priority: 26.02.85 US 705820

(43) Date of publication of application:
24.09.86 Bulletin 86/39

(84) Designated Contracting States:
BE DE FR GB IT NL SE

(71) Applicant: MOBIL OIL CORPORATION
150 East 42nd Street
New York New York 10017(US)

(72) Inventor: Valyocsik, Ernest William
960 Randolph Drive
Yardley Pennsylvania 19067(US)

(74) Representative: West, Alan Harry
Mobil Court 3 Clements Inn
London WC2A 2EB(GB)

## (54) Tropane derivatives, their synthesis and their use.

(57) This invention is directed to novel quaternary ammonium tropane compounds and 3-substituted derivatives thereof and their synthesis. The compounds are novel compounds having valuable utility as organic directing agents in the crystallization of various silicates, which, in turn, are useful as catalyst components, sorbents, and/or ion-exchangers. More particularly, this invention is concerned with quaternary ammonium tropane compounds and 3-substituted derivatives thereof synthesized by selective alkylation of the bridgehead nitrogen of tropane or 3-substituted derivatives thereof.

EP 0 195 498 A2

# TROPANE DERIVATIVES, THEIR SYNTHESIS AND THEIR USE

This invention relates to tropane derivatives, their synthesis and their use.

Tropane, or 8-methyl-8-azabicyclo[3.2.1]octane, having the formula (I) wherein R' and R" are both hydrogen, is a known compound detailed in The Merck Index, 9th Ed., No. 9440. A number of substituted derivatives of tropane are also detailed in The Merck Index, 9th Ed., as follows:

Tropine, No. 9446 or endo-8-methyl-8-azabicyclo-[3.2.1]-octan-3-ol, has the above formula wherein R' is hydrogen and R" is hydroxide and can be prepared as in U.S. Patents 2,366,760 and 2,746,976.

Tropentane, No. 9442 or 1-phenylcyclopentanecarboxylic acid 8-methyl-8-azabicyclo[3.2.1]oct-3-yl ester, has the above formula wherein R' is hydrogen and R" is of the formula (II) and is prepared from tropine and 1-phenylcyclopentanecarboxyl chloride.

Tropeine, No. 9441 is a name given esters of tropine in general, while tropine benzylate, No. 9447 or endo-alpha-hydroxy-alpha-phenylbenzeneacetic acid 8-methyl-8-azabicyclo[3.2.1]oct-3-yl ester, has the above formula wherein R' is hydrogen and R" is of the formula (III).

Tropacine, No. 9436 or endo-alpha-phenylbenzeneacetic acid 8-methyl-8-azabicyclo[3.2.1]oct-3-yl ester, has the above formula wherein R' is hydrogen and R" is of the formula (IV) and is prepared from tropine and diphenylacetyl chloride as described in Swiss Patent 202,181.

Atropine, No. 892 or endo-(±)-alpha-(hydroxymethyl)-benzeneacetic acid 8-methyl-8-azabicyclo[3.2.1]oct-3-yl ester, has the above formula wherein R' is hydrogen and R" is of the formula (V).

According to one aspect of the present invention, there is provided a quaternary ammonium tropane compound having the formula (VI) wherein R is alkyl of 1 to 10 carbon atoms, R' and R" are groups selected from formulae I, II, III and IV above, hydrogen, hydroxide, alkyl of 1 to 10 carbon atoms alkenyl of 2 to 8 carbon atoms, ester (such as methyl acetate, ethyl acetate, n-propyl acetate, ethyl formate and ethyl propionate), ether (such as dimethyl ether and diethyl ether) and combinations thereof, and X is an anion selected from halide, (e.g. iodide, chloride or bromide), hydroxide, nitrate, perchlorate and bisulfate.

According to a further aspect, the invention resides in a method for synthesizing a compound having the formula (VI) wherein R is alkyl of 1 to 10 carbon atoms, R' and R" are radicals selected from those of formulae (II), (III), (IV) and (V), hydrogen, hydroxyl, alkyl of 1 to 10 carbon atoms, alkenyl of 2 to 8 carbon atoms, ester, ether and combinations thereof, and X is an anion, which comprises forming a reaction mixture comprising from 5 to 20 weight percent tropane or a derivative thereof substituted at the 3 position by said radical(s), from 10 to 20 weight percent of an alkylating agent having 1 to 10 carbon atoms, and from 60 to 85 weight percent of a solvent, and maintaining said mixture at reaction conditions of temperature and pressure sufficient to permit synthesis of said compound.

According to yet a further aspect, the invention resides in a method of preparing a synthetic crystalline silicate material comprising a reaction mixture containing sources of an alkali metal or alkaline earth metal oxide, an oxide of silicon, an oxide of aluminum, water and a quaternary ammonium tropane compound or 3-substituted derivative thereof having the formula (VI) wherein R is alkyl of 1 to 10 carbon atoms, R' and R" are radicals selected from those of formulae (II), (III), (IV) and (V), hydrogen, hydroxyl, alkyl of 1 to 10 carbon atoms, alkenyl of 2 to 8 carbon atoms, ester, ether and combinations thereof and X is an anion, and maintaining said reaction mixture under sufficient crystallization conditions until crystals of said silicate material are formed.

As will be noted from the above formula (VI) for the quaternary ammonium tropane compounds and 3-substituted derivatives thereof of this invention, the present compounds are synthesized by alkylation selectively at the bridgehead nitrogen of the starting material compound, examples of which are tropane, tropine, tropentane, tropine benzylate, tropacine and atropine. The R' and R" substituents may be the same of different, although preferably at least one of the substituents is hydrogen. Most preferably R' is hydrogen and R" is hydroxyl.

Non-limiting examples of the compounds of this invention include compounds of methyltropinium, ethyltropinium, propyltropinium, butyltropinium, pentyltropinium, hexyltropinium, heptyltropinium, octyltropinium, nonyltropinium and decyltropinium. The alkyl substituent, R, may be linear or branched, with linear preferred.

The synthesis method utilized for preparation of the compounds of this invention involves contacting the appropriate starting material compound with an alkylating agent dissolved in a suitable solvent medium at reaction conditions including a temperature of from about ambient to reflux, and a pressure of from 100 to 3549 kPa - (atmospheric to 500 psig), preferably about 100 kPa - (atmospheric). The time required for the reaction will, of course, depend upon such factors as temperature and pressure, as well as relative concentrations of reactants and desired degree of reaction, but usually will be from 6 hours to 4 days. Generally, the reaction mixture will comprise from 5 to 20 weight percent tropane or 3-substituted derivative thereof, from 10 to 20 weight percent alkylating agent and from 60 to 85 weight percent solvent.

The solvent is preferably polar and is suitably an alcohol of 1 to 8 carbon atoms, an ether of 2 to 10 carbon atoms, or a combination thereof, especially methanol, ethanol, acetone and/or diethyl ether. The tropane or 3-substituted derivative thereof should be soluble in the solvent chosen to at least about 20 percent. The preferred solvent will be essentially water-free, as will the reaction mixture in which the present compounds are produced. Choice of solvent will determine to a substantial degree the reflux temperature at which the reaction will proceed to produce the required quaternary ammonium tropane compound or 3-substituted derivative thereof. For example, for an ethanol solvent, the reflux temperature will be about 70°C, whereas for a methanol solvent the reflux temperature will be about 65°C.

The alkylating agent may be an olefin such as, for example, ethylene, propylene, butene, decene, formaldehyde, an alkyl halide or an alcohol, provided the alkyl portion thereof has from 1 to 10 carbon atoms. Numerous other acyclic compounds able to provide at least one reactive $C_1$ to $C_{10}$ alkyl radical may be utilized as alkylating agents. The preferred alkylating agent is alkyl halide of from 1 to 10 carbon atoms, especially the chloride, bromide or iodide, with iodide preferred. When an alkyl halide is used, the halide moiety will of course be the anion of the compound of the invention.

Compounds of above formula (VI) are useful as directing agents in the synthesis of crystalline silicates. Such silicates are useful as catalyst components, sorbents and/or ion exchangers. Such silicates are prepared from a reaction mixture containing sources of an alkali or alkaline earth

metal oxide, an oxide of aluminum, an oxide of silicon, water and the directing agent compound, as is exemplified hereinafter. Such reaction mixture will have a composition, in terms of mole ratios of oxides, within the following ranges:

| | |
|---|---|
| $SiO_2/Al_2O_3$ | 50-1000 |
| $H_2O/SiO_2$ | 5-200 |
| $OH^-/SiO_2$ | 0-2.0 |
| $M/SiO_2$ | 0.01-3.0 |
| $R'''/SiO_2$ | 0.01-2.0 |

wherein R"' is the cation of the directing agent compound - (VI) and M is the alkali or alkaline earth metal cation.

Crystallization of the silicate can be carried out under either static or stirred condition in a suitable reactor vessel, such as for example, polypropylene jars or teflon lined or stainless steel autoclaves. The temperature range for the silicate crystallization is generally from 100°C to 200°C for a time sufficient for crystallization to occur at the temperature used, e.g. from 48 hours to 15 days. Thereafter, the crystals are separated from the liquid and recovered. The reaction mixture can be prepared utilizing materials which supply the appropriate oxides. Such materials may include sodium silicate, silica hydrosol, silica gel, silicic acid, sodium hydroxide, a source of aluminum, and the directing agent compound.

It should be realized that the reaction mixture oxides can be supplied by more than one source. The reaction mixture can be prepared either batchwise or continuously. Crystal size and crystallization time of the crystalline silicate material will vary with the nature of the reaction mixture employed and the crystallization conditions.

The following examples illustrate the present invention.

Examples 1-5

Alkyltropinium halides, i.e., iodides, were prepared by reacting tropine with the appropriate alkyliodide in absolute ethanol according to the reaction (VII), where R was individually methyl, ethyl, propyl, butyl and pentyl.

The reaction mixture was generally refluxed for 1-3 days before quenching the reaction vessel in a dry ice-acetone bath to -20°C. The crystalline product was separated from the solvent and filtered on a Büchner funnel. The crystals were dried in an air stream, then chemically analyzed. Table 1 records the analytical results for the series of alkyltropinium iodides synthesized in these examples.

Table 1

| Example | Product Compound | Compositions wt.% C | H | N | O | I |
|---|---|---|---|---|---|---|
| 1 | Methyltropinium Iodide | 38.33 | 6.51 | 4.87 | 5.6 | 44.74 |
| 2 | Ethyltropinium Iodide | 40.38 | 6.81 | 4.58 | 5.2 | 42.38 |
| 3 | Propyltropinium Iodide | 42.16 | 6.99 | 4.41 | 5.1 | 40.50 |
| 4 | Butyltropinium Iodide | 44.27 | 7.58 | 4.63 | 3.9 | 39.58 |
| 5 | Pentyltropinium Iodide | 44.90 | 7.71 | 4.12 | 5.8 | 37.47 |

The following examples show utility of the quaternary ammonium tropane compounds and 3-substituted derivatives thereof of this invention as crystallization directing agents in manufacture of synthetic crystalline silicates. In the examples, whenever adsorption data are set forth for comparison of sorptive capacities for water, cyclohexane and/or n-hexane, they were determined as follows:

A weighed sample of the calcined crystalline silicate adsorbant was contacted with the desired pure adsorbate vapor in an adsorption chamber, evacuated to less than 1 mm and contacted with 12 mm Hg of water vapor or 20 mm Hg of n-hexane or cyclohexane vapor, pressures less than the vapor-liquid equilibrium pressure of the respective adsorbate at room temperature. The pressure was kept constant (within about ± 0.5mm) by addition of adsorbate vapor controlled by a manostat during the adsorption period, which did not exceed about 8 hours. As adsorbate was adsorbed by the silicate absorbant, the decrease in pressure caused the manostat to open a valve which admitted more adsorbate vapor to the chamber to restore the above control pressures. Sorption was complete when the pressure change was not sufficient to activate the manostat. The increase in weight was calculated as the adsorption capacity of the sample in g/100 g of calcined adsorbant.

When Alpha Value of the synthesized crystalline silicate is examined, it is noted that the Alpha Value is an approximate indication of the catalytic cracking activity of a catalyst compared to a standard catalyst and gives the relative rate constant (rate of normal hexane conversion per volume of catalyst per unit time). It is based on the activity of the highly active silica-alumina cracking catalyst taken as an Alpha of 1 (Rate Constant = 0.016 $sec^{-1}$). The Alpha Test is described in U.S. Patent 3,354,078 and in The Journal of Catalysis, Vol. IV, pp. 522-529 (August 1965). It is noted that intrinsic rate constants for many acid-catalyzed reactions are proportional to the Alpha Value for a particular crystalline silicate catalyst, i.e. the rates for toluene disproportionation, xylene isomerization, alkene conversion and methanol conversion (see "The Active Site of Acidic Aluminosilicate Catalysts,"Nature, Vol. 309, No. 5969, pp. 589-591, 14 June 1984).

Examples 6 -11

Six separate synthesis reaction mixtures were prepared with compositions indicated in Table 2. The mixtures were prepared with silica sol (30 percent $SiO_2$), $NaAlO_2$, NaOH, water, and a compound of the present invention as a directing agent, i.e. methyltropinium iodide prepared in Example 1. The mixtures were maintained at 160°C for 4 days in a stainless steel, stirred (400 rpm) autoclave. Solids were separated from any unreacted components by filtration and then water washed, followed by drying at 110°C. The product crystals were analyzed by X-ray diffraction and chemical analysis. The product of Example 6 was found to be a crystalline silicate having a particular X-ray diffraction pattern with a trace of unidentified second component impurity. The products from Examples 7-11 proved to be 100 percent crystalline silicate having the same X-ray diffraction pattern as that of Example 6. The crystalline silicate of Examples 6-11, designated ZSM-58, is described in more detail in our copending Application No.____(corresponding to U.S. Patent Application No. 705821, Mobil Docket F-3364).

The X-ray diffraction pattern of the Example 9 crystals, after calcination at 538°C for 17 hours in air, is set forth as illustration in Table 3. Other properties of each crystalline product are presented in Table 4. In the latter table, compositions are calculated on the basis of 100 ($SiO_2$ + $AlO_2^-$) tetrahedra. The as-synthesized crystalline materials contained from 3.8 to 5.0 methyltropinium cations per 100 tetrahedra.

Table 2

| Example | Mixture Composition, Mole Ratio | | | | |
|---|---|---|---|---|---|
| | $SiO_2/Al_2O_3$ | $H_2O/SiO_2$ | $OH^-/SiO_2$ | $Na^+/SiO_2$ | $R^*/SiO_2$ |
| 6 | 300 | 40 | 0.30 | 0.31 | 0.25 |
| 7 | 200 | 40 | 0.30 | 0.31 | 0.25 |
| 8 | 90 | 40 | 0.40 | 0.42 | 0.25 |
| 9 | 90 | 40 | 0.30 | 0.32 | 0.25 |
| 10 | 90 | 40 | 0.30 | 0.32 | 0.25 |
| 11 | 70 | 40 | 0.30 | 0.33 | 0.25 |

*R = methyltropinium cation.

Table 3

| d(A) | Observed 2 Theta | Relative Intensity |
|---|---|---|
| 13.57425 | 6.511 | 7.4 |
| 11.44933 | 7.721 | 51.2 |
| 10.29541 | 8.588 | 4.1 |
| 7.76959 | 11.389 | 53.6 |
| 6.89736 | 12.834 | 60.1 |
| 6.84556 | 12.932 | 33.0 |
| 6.15999 | 14.378 | 57.8 |
| 5.91115 | 14.987 | 19.5 |
| 5.74071 | 15.435 | 85.8 |
| 5.16339 | 17.173 | 100.0 |
| 4.84326 | 18.317 | 51.9 |
| 4.70389 | 18.865 | 56.0 |
| 4.52632 | 19.612 | 20.3 |
| 4.49392 | 19.755 | 51.7 |
| 4.41905 | 20.093 | 4.7 |
| 4.13559 | 21.486 | 26.0 |
| 3.98517 | 22.307 | 11.8 |
| 3.96826 | 22.404 | 8.9 |
| 3.87191 | 22.969 | 17.1 |
| 3.82281 | 23.268 | 30.6 |
| 3.80712 | 23.365 | 25.6 |
| 3.57841 | 24.882 | 16.2 |
| 3.44668 | 25.849 | 35.2 |
| 3.38811 | 26.303 | 96.5 |
| 3.35769 | 26.546 | 86.7 |
| 3.34862 | 26.619 | 80.8 |
| 3.30859 | 26.947 | 66.2 |
| 3.28346 | 27.158 | 9.1 |
| 3.16039 | 28.237 | 23.3 |
| 3.06246 | 29.159 | 26.8 |
| 3.06070 | 29.176 | 31.2 |
| 3.03737 | 29.406 | 22.7 |
| 2.99654 | 29.816 | 25.2 |
| 2.98814 | 29.901 | 21.2 |
| 2.87045 | 31.158 | 4.1 |
| 2.84237 | 31.473 | 5.1 |
| 2.66429 | 33.638 | 5.5 |
| 2.58922 | 34.643 | 4.8 |
| 2.50349 | 35.869 | 4.3 |
| 2.48809 | 36.099 | 6.3 |
| 2.43821 | 36.863 | 9.0 |
| 2.42105 | 37.134 | 14.9 |

Table 3 (continued)

| d(A) | Observed 2 Theta | Relative Intensity |
|---|---|---|
| 2.39052 | 37.626 | 5.8 |
| 2.35412 | 38.230 | 2.8 |
| 2.33296 | 38.591 | 4.3 |
| 2.30029 | 39.161 | 16.7 |
| 2.23686 | 40.319 | 2.4 |
| 2.23188 | 40.413 | 1.9 |
| 2.21126 | 40.807 | 3.2 |
| 2.16400 | 41.739 | 1.7 |
| 2.11106 | 42.836 | 1.4 |
| 2.07314 | 43.660 | 3.0 |
| 2.03910 | 44.427 | 0.3 |
| 1.97783 | 45.880 | 11.4 |
| 1.95022 | 46.568 | 4.4 |
| 1.93214 | 47.030 | 3.9 |
| 1.91503 | 47.476 | 3.7 |
| 1.83810 | 49.594 | 6.4 |
| 1.83554 | 49.667 | 5.5 |

Table 4

| Example | Moles C / Mole N | Moles per Mole $Al_2O_3$: $N_2O$ | $Na_2O$ | $SiO_2$ |
|---|---|---|---|---|
| 6 | 9.5 | 4.09 | 0.85 | 223 |
| 7 | 11.2 | 2.43 | 0.74 | 140 |
| 8 | 9.6 | 1.85 | 0.13 | 83 |
| 9 | 10.2 | 1.69 | 0.12 | 78 |
| 10 | 10.8 | 1.77 | 0.25 | 85 |
| 11 | 9.6 | 1.50 | 0.10 | 62 |

Table 4 (continued

| Example | COMPOSITION | | | |
|---|---|---|---|---|
| | Al | $Na^+$ | $N^+$ | R |
| | $100T_d$ | $100T_d$ | $100T_d$ | $100T_d$ |
| 6 | 0.89 | 0.76 | 3.6 | 3.8 |
| 7 | 1.4 | 1.0 | 3.4 | 4.2 |
| 8 | 2.4 | 0.30 | 4.4 | 4.7 |
| 9 | 2.5 | 0.30 | 4.2 | 4.8 |
| 10 | 2.3 | 0.58 | 4.1 | 4.9 |
| 11 | 3.1 | 0.30 | 4.7 | 5.0 |

Example 12

A sample of the Example 9 product crystals, having been calcined in nitrogen for 4 hours at 500°C, ammonium exchanged and converted to the hydrogen form, was subjected to the sorption test. Significant n-hexane, i.e. 8 weight percent at 90°C, was sorbed while only minimal cyclohexane (about 1 weight percent at 90°C) sorbed at 80 torr hexane partial pressure. This indicates molecular shape selectivity, a very valuable property, for the crystalline silicate directed by the methyltropinium iodide of Example 1.

Example 13

The sample of Example 9 product used for sorption evaluation was evaluated in the Alpha Test. Its Alpha Value proved to be 13 at 538°C.

Examples 14-21

Eight additional separate synthesis mixtures were prepared with compositions indicated in Table 5. The mixtures were prepared with silica sol (30% $SiO_2$), $NaAlO_2$, NaOH, water and an alkyltropinium iodide, where the alkyl group is, individually, ethyl, n-propyl, n-butyl and n-pentyl (as synthesized in Examples 2-5). The mixtures were maintained at 160°C for 4 days in a stirred (400 rpm) stainless steel autoclave at autogenous pressure. At the termination of the synthesis (4 days in each), the solids were separated from unreacted components by filtration, water washed, then dried at 110°C. The solid products were analyzed by x-ray diffraction and chemical analysis. The solid crystalline silicate products of Examples 14-21 were of zeolite ZSM-5 structure see (U.S. Patent 3,702,886 of varying crystallinity, as recorded in Table 5. The product of Example 17 also contained alpha-quartz.

Results of chemical analyses are presented in Table 6.

Table 5

| Example | Directing Agent* | Mixture Composition (mole ratios) $SiO_2/Al_2O_3$ | $H_2O/SiO_2$ | $OH^-/SiO_2$ |
|---|---|---|---|---|
| 14 | Ethyltropinium | 60 | 40 | 0.30 |
| 15 | n-Propyltropinium | 70 | 40 | 0.30 |
| 16 | n-Propyltropinium | 90 | 40 | 0.30 |
| 17 | n-Propyltropinium | 200 | 40 | 0.30 |
| 18 | n-Butyltropinium | 60 | 40 | 0.30 |
| 19 | n-Butyltropinium | 180 | 40 | 0.30 |
| 20 | n-Pentyltropinium | 90 | 40 | 0.30 |
| 21 | n-Pentyltropinium | 180 | 40 | 0.30 |

Table 5 (continued)

| Example | Directing Agent* | Mixture Composition (mole ratios) $Na^+/SiO_2$ | $R^{**}/SiO_2$ | Product Crystallinity |
|---|---|---|---|---|
| 14 | Ethyltropinium | 0.33 | 0.20 | 75% |
| 15 | n-Propyltropinium | 0.33 | 0.23 | 70% |
| 16 | n-Propyltropinium | 0.32 | 0.20 | 75% |
| 17 | n-Propyltropinium | 0.31 | 0.20 | 70% |
| 18 | n-Butyltropinium | 0.33 | 0.15 | 90% |
| 19 | n-Butyltropinium | 0.31 | 0.15 | 100% |
| 20 | n-Pentyltropinium | 0.32 | 0.15 | 50% |
| 21 | n-Pentyltropinium | 0.31 | 0.15 | 80% |

* Iodide Salt
**R = the alkyltropinium cation

Table 6

| Example | Moles C / Mole N | Moles per Mole $Al_2O_3$: $N_2O$ | $Na_2O$ | $SiO_2$ |
|---|---|---|---|---|
| 14 | 12.4 | 1.0 | n.d. | 61 |
| 15 | 11.4 | 1.4 | 0.10 | 66 |
| 16 | 11.4 | 1.7 | n.d. | 83 |
| 17 | 11.6 | 2.9 | 0.28 | 195 |
| 18 | 12.9 | 1.2 | 0.46 | 60 |
| 19 | 12.9 | 3.3 | 1.0 | 198 |
| 20 | 14.0 | 1.4 | 1.4 | 97 |
| 21 | 13.8 | 3.0 | 0.83 | 186 |

**Claims**

1. A compound having the formula (VI) wherein R is alkyl of from 1 to 10 carbon atoms, R' and R" are groups selected from those of formulae I, II, III and IV, hydrogen, hydroxyl, alkyl of 1 to 10 carbon atoms, alkenyl of 2 to 8 carbon atoms, ester, ether and combinations thereof, and X is an anion.

2. The compound of Claim 1 wherein the anion X is selected from halide, hydroxide, nitrate, perchlorate and bisulfate.

3. The compound of Claim 1 or Claim 2 wherein R is methyl, ethyl, propyl, butyl or pentyl.

4. The compound of any preceding Claim wherein R' is hydrogen.

5. The compound of any preceding Claim wherein R" is hydroxyl.

6. The N-methytropinium cation.

7. A method for synthesizing a compound having the formula (VI) wherein R is alkyl of 1 to 10 carbon atoms, R' and R" are radicals selected from those of formulae (II), -(III) and (IV) and (V), hydrogen, hydroxyl, alkyl of 1 to 10 carbon atoms, alkenyl of 2 to 8 carbon atoms, ester, ether and combinations thereof, and X is an anion, which comprises forming a reaction mixture comprising from 5 to 20 weight percent tropane or a derivative thereof substituted at the 3-position by said radical(s), from 10 to 20 weight percent of an alkylating agent having 1 to 10 carbon atoms and from 60 to 85 weight percent of a solvent, and maintaining said mixture at reaction conditions of temperature and pressure sufficient to permit synthesis of said compound.

8. The method of Claim 7 wherein said alkylating agent is selected from olefins of 1 to 10 carbon atoms, formaldehyde, alkyl halides of 1 to 10 carbon atoms and alcohols of 1 to 10 carbon atoms, and said solvent is an alcohol of 1 to 8 carbon atoms, an ether of 2 to 10 carbon atoms or a combination thereof.

9. The method of Claim 7 or Claim 8 wherein said reaction conditions include a temperature of ambient to reflux and a pressure of 100 to 3549 kPa (atmospheric to 500 psig).

10. A method of preparing a synthetic crystalline silicate material comprising preparing a reaction mixture containing sources of an alkali metal or alkaline earth metal oxide, an oxide of silicon, an oxide of aluminum, water and a quaternary ammonium tropane compound or 3-substituted derivative thereof directing agent of the formula (VI) wherein R is alkyl of 1 to 10 carbon atoms, R' and R" are radicals selected from those of formulae (II), (III), (IV) and (V), hydrogen, hydroxyl, alkyl of 1 to 10 carbon atoms, alkenyl of 2 to 8 carbon atoms, ester, ether and combinations thereof and X is an anion, and maintaining said reaction mixture under sufficient crystallization conditions until crystals of said silicate material are formed.

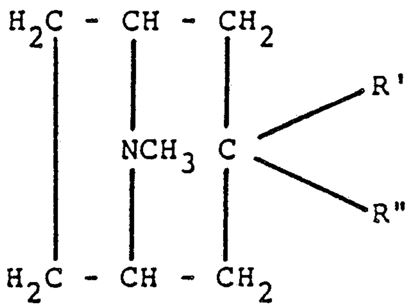

(I)

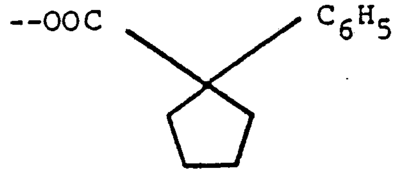

(II)

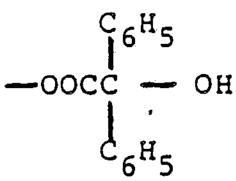

(III)

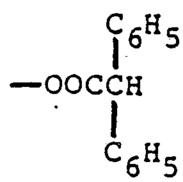

(IV)

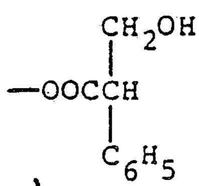

(V)

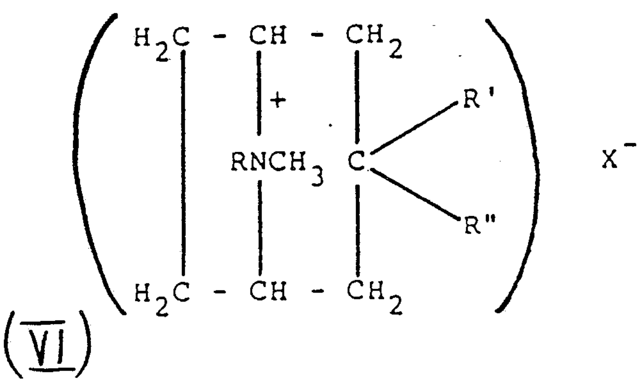

(VI)

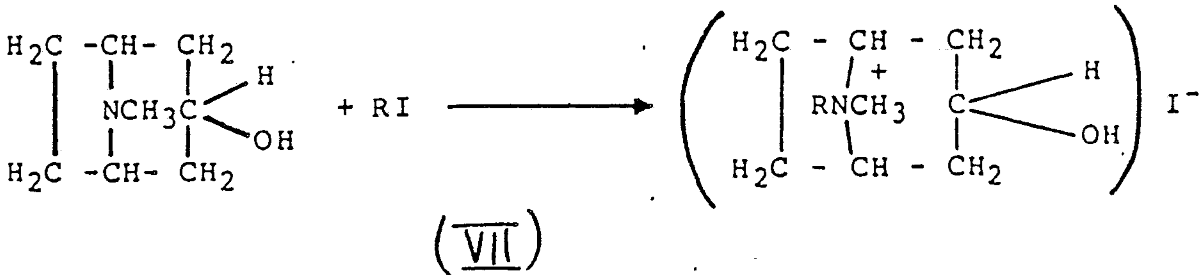

(VII)